# EUROPEAN PATENT SPECIFICATION

(11) **EP 3 672 646 B1**
(45) Date of publication and mention of the grant of the patent: **05.03.2025**
(21) Application number: 18848614.6
(22) Date of filing: 21.08.2018
(51) Int. Cl.: A61B 50/30, A61B 50/33, B65D 65/10, B65D 75/14

(54) **STERILISATION WRAP AND METHOD**
STERILISATIONSHÜLLE UND VERFAHREN
ENVELOPPE DE STÉRILISATION ET MÉTHODE

(30) Priority: 21.08.2017 AU 2017101130
(43) Date of publication of application: 01.07.2020
(73) Proprietor: Multigate Medical Products Pty Ltd, Villawood, New South Wales 2163 (AU)
(72) Inventor: LEEDHAM, Amy, Villawood, NSW 2163 (AU); CHANG, Peter, Villawood, NSW 2163 (AU)
(74) Representative: Cummings, Sean Patrick
(86) International application number: PCT/AU2018/000143
(87) International publication number: WO 2019/036743

(56) References cited:
- WO-A2-2013/046187
- WO-A2-2013/046187
- DE-A1- 102008 035 279
- DE-A1- 102012 006 445
- JP-A- 2002 362 625
- JP-A- 2002 362 625
- US-A1- 2007 026 472
- US-A1- 2015 307 223

## Description

### Technical Field

This invention is concerned with a new wrap and method for sterilization packs used in hospitals, clinics and similar places. In particular, the invention relates to a wrap for sterilization packs, often of relatively small dimensions, usually having single-use contents, and methods for using the wrap.

For convenience, the invention will often be described below in relation to a wrap for an object to be wrapped being a single use tray. However, it is to be understood that the invention is not limited to a particular object.

### Background of the Invention

At present, single use trays containing dressings and/ or other items are provided to users wrapped in a sterilization wrap and sealed in a plastic film after sterilizing. The user removes the plastic film, unwraps the sterilization wrap and uses it as an aseptic surface beneath the tray.

For single use trays and similar objects, the wrap usually has dimensions of up to about 1 metre by 1 metre. For a small tray, the dimensions are usually about 500 mm x 500 mm. The material used in such small wraps is usually a very lightweight plastic, having a weight of up to 45 gsm and preferably about 25 gsm.

The wrap is regarded as aseptic provided the proper procedure has been followed for unwrapping.

So that the wrap can be unfolded to provide an aseptic surface, at present the wrap is folded around the contents with at least one corner protruding.

Users such as nurses, clinicians and doctors are trained to open such a wrap by carefully pulling out the protruding corner, lying that part of the wrap flat (if possible), locating the next corner, carefully pulling it out and lying the wrap part flat, and repeating these actions until the contents are fully unwrapped. At this stage, the object is located on the wrap and ready for use once the user dons gloves.

During the unwrapping process, a user must take care not to touch the inside of the wrap. The user unfolds the wrap slowly in order to ensure that the user does not accidentally touch the aseptic field. In some cases, one or more corners of the wrap are not readily accessible and the user must locate each such corner, tease it away from the package and take hold of it without compromising asepsis.

As a result, significant time can be spent on properly unfolding a sterile wrapped object or the user will routinely compromise asepsis by accidentally touching the inside of the wrap.

In another example, document JP2002362625 describes a wrapping material equipped with at least one catch affixed to an external surface of a sheet with a predetermined size.

WO 2013/046187 A2 describes a flexible multi-panel sterilisation assembly for sterilising surgical products, the barrier panel comprising side tabs.

It is an object of the present invention to provide an improved wrap which, at least in some embodiments, enables an object to be unwrapped much more efficiently that previously possible while maintaining asepsis of the object and the aseptic field of the wrap.

### Disclosure of the Invention

The invention is defined in the independent claims and other embodiments are listed in the dependent claims.

Accordingly, the present invention provides a wrap for wrapping an object to be sterilised, the wrap having four edges bound by a periphery, with a first edge on one side of the periphery and a second edge on a side of the periphery opposite to the first side, wherein the wrap has at least a first tab forming part of the periphery of the first edge and at least a second tab forming part of the periphery of the second edge, the first tab being diagonally opposite from the second tab.

The invention also includes a method of wrapping an object to be sterilised using the wrap of the invention, the method including the steps of:
(a) positioning the object on the wrap so that the object is located between the first edge and the second edge;
(b) folding the wrap at a first fold line a small distance inboard of the first edge so that the first tab is under the wrap;
(c) folding the wrap at a second fold line, inboard of the first fold line, towards the object so that the object is minimally covered by the wrap;
(d) folding the wrap at a third fold line a small distance inboard of the second edge so that the second tab is under the wrap;
(e) folding the wrap at a fourth fold line, inboard of the third fold line, towards the object so that the object is further covered by the wrap; and
(f) folding the wrap in a direction substantially perpendicular to the first to fourth fold lines.

Optionally, a step may be added between step (b) and step (c), namely:
(b1) folding the wrap towards the object at an optional fold line a small distance inboard of the first fold.

Step (b1) may be taken when the object is small and may be repeated as many times as necessary. Preferably, the or each optional fold line is of a similar distance to that between the first fold line and the first edge. An example of such a distance is 2.5 to 5cm

The invention also provides a method of unwrapping the wrapped object, the method including the steps of:
(a) removing or tearing any sticker holding the wrap around the wrapped object;
(b) unfolding the wrap in directions substantially perpendicular to the first fold line;
(c) using the or each second tab to pull the wrap away from the object; and
(d) using the or each first tab to pull the wrap away from the object.

The wrap of the invention is preferably rectilinear and of a relatively small size, being up to about 1 metre x 1 metre in dimensions. A preferred size is about 500 x 500 mm.

The material of the wrap is preferably the same type of lightweight plastic currently in use for wrapping small disposable objects such as trays, of up to 45 gsm.

The wrap if rectilinear may have square corners, but preferably the corners are rounded.

The first tab is located diagonally opposite the second tab. In an especially-preferred embodiment, there are two tabs at the first edge and two tabs at the second edge, the tabs at the first edge being spaced apart and located opposite the tabs at the second edge.

Each tab may be of any suitable shape. In a preferred embodiment, each tab has parallel sides and a rounded end.

The tabs may be integral with the wrap or may be added to the wrap in any suitable way, including using adhesive or spot welding.

The wrap preferably has a length and width which is at least four times the length and width of the object. For example, if the object measures about 100 x 100 mm, the wrap (excluding tabs) is a minimum of 400 x 400 mm. This enables the object to be wrapped in several layers, as illustrated in the examples shown in the drawings.

In the method of the invention, the object to be wrapped is preferably positioned on the wrap according to the size of the object. If, for example, the object is small, it is preferably positioned about midway between the edges of the wrap which do not have any tabs, but closer to second tabbed edge, such as inboard of the second edge by about the length of the object plus about 2 cm.

If the object is larger, it is preferably positioned on the wrap about midway between the first and second edges, but closer to an edge of the wrap which does not have any tabs, such as inboard of the untabbed edge by about the width of the object plus about 2 cm.

Step (f) of the folding method may be repeated three times or more. The folded wrap may be secured by a sticker if desired.

The folded object, after sterilisation, may be sealed in plastic film.

### Brief Description of the Drawings

The wrap and method of the invention may be better understood from the following nonlimiting description of preferred embodiments. In the drawings:
Figure 1 is a plan view of an embodiment of a wrap according to the invention, with a large object in position, ready for wrapping before sterilisation;
Figure 2 shows the wrap of Figure 1, with the first fold indicated;
Figure 3 shows the embodiment in Figures 1 and 2, after the wrap has been folded at the first fold and indicating the location of the second fold;
Figure 4 shows the embodiment after the wrap has been folded at the second fold and indicating the location of the third fold;
Figure 5 illustrates the embodiment after the wrap has been folded at the third fold and indicating the location of the fourth fold;
Figure 6 shows the embodiment after the wrap has been folded at the fourth fold;
Figure 7 shows the wrap of Figure 6, indicating the location of the first perpendicular fold;
Figure 8 shows the wrap after folding at the first perpendicular fold and indicating the second perpendicular fold;
Figure 9 shows the wrap after folding at the second perpendicular fold and indicating the third perpendicular fold;
Figure 10 shows the wrap after it has been folded at the third perpendicular fold and secured with respect to the wrapped object;
Figure 11 is a plan view of a second embodiment of a wrap according to the invention, with a small object in position, ready for wrapping before sterilisation;
Figure 12 shows the wrap of Figure 11, with the first fold indicated;
Figure 13 shows the embodiment in Figures 11 and 12, after the wrap has been folded at the first fold and indicating the location of a first optional fold;
Figure 14 shows the wrap of Figure 12, after folding at the first optional fold and indicating the location of a second optional fold;
Figure 15 shows the embodiment after the wrap has been folded at the second optional fold and indicating the location of the second fold;
Figure 16 illustrates the embodiment after the wrap has been folded at the second fold and indicating the location of the third fold;
Figure 17 shows the embodiment after the wrap has been folded at the third fold and indicating the location of the fourth fold;
Figure 18 shows the wrap of Figure 17, after folding at the fourth fold and indicating the location of two first perpendicular folds;
Figure 19 shows the wrap after folding at the first perpendicular folds and indicating the second perpendicular fold;
Figure 20 shows the wrap after folding at the second perpendicular fold and indicating the third perpendicular fold; and
Figure 21 shows the wrap after it has been folded at the third perpendicular fold and secured with respect to the wrapped object.

### Detailed Description of the Drawings

Possible and preferred features of the present invention will now be described with particular reference to the accompanying drawings. However, it is to be understood that the features illustrated in and described with reference to the drawings are not to be construed as limiting on the scope of the invention.

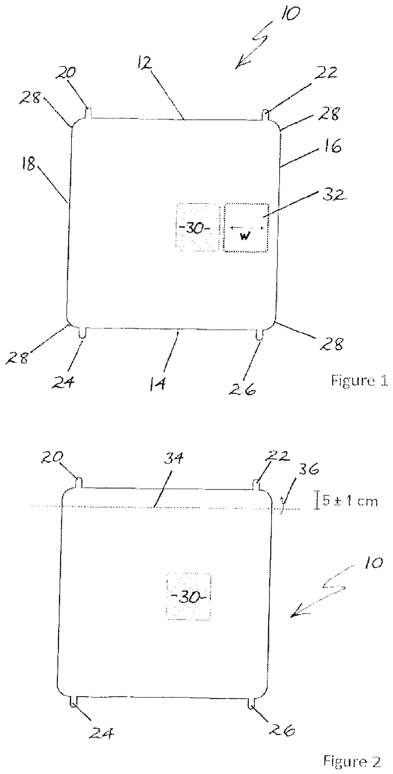

Referring first to Figure 1, wrap 10 has a periphery consisting of first edge 12, second edge 14, third edge 16 and fourth edge 18. There are two tabs, 20 and 22, on the first edge 12 and two tabs 24 and 26 on the second edge 14, which is opposite to the first edge 12. Tab 20 is opposite tab 24, while tab 22 is opposite tab 26.

Wrap 10 has a generally square shape (excluding tabs 20, 22, 24 and 26). In this embodiment, wrap 10 has rounded corners 28. It is to be appreciated that wrap 10 may have square corners instead.

In this embodiment, wrap 10 is a single sheet made of a lightweight plastic material, weighing approximately 25 gsm and measuring about 500 mm by 500 mm.

Plastic disposable tray 30 is placed on the wrap 10 as shown in Figure 1. Tray 30 may bear dressings, disposable forceps and the like but these are not shown. Tray 30 is located approximately halfway between first edge 12 and second edge 14. Tray 30 is also located about one tray length 32 inboard of third edge 16.

In this embodiment, tray 30 is regarded as large and is 130mm long and 140mm wide. Tray 30 is placed on wrap 10 approximately centrally between first edge 12 and second edge 14. Tray 30 is inboard of edge 16 by about the width W of tray 30.

Figure 2 shows first fold line 34, which is a small distance (between about 4 and 6 cm) inboard of first edge 12. Wrap 10 is folded under, in the direction of arrow 36, at first fold line 34, so that tabs 20 and 22 are under wrap 10, and the fold is flattened.

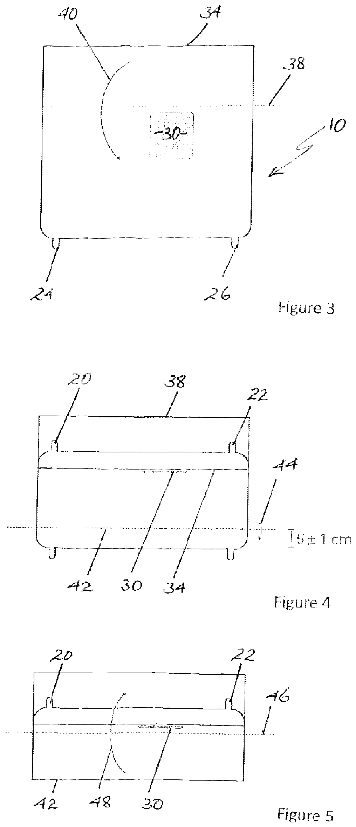

The result is shown in Figure 3. Wrap 10 is then folded at second fold line 38, towards tray 30, in the direction of arrow 40. Second fold line 38 is located so that after folding tray 30 will be only just covered by wrap 10.

The result is shown in Figure 4. Tabs 20 and 22 are now visible. Wrap 10 is then folded at third fold line 42, which is a small distance (between about 4 and 6 cm) inboard of second edge 14 towards tray 30, in the direction of arrow 44, so that tabs 24 and 26 are under wrap 10. The fold is flattened.

The result is shown in Figure 5. Wrap 10 is then folded at fourth fold line 46, towards tray 30, in the direction of arrow 48. Fourth fold line 46 is located so that after folding tray 30 is further covered by wrap 10.

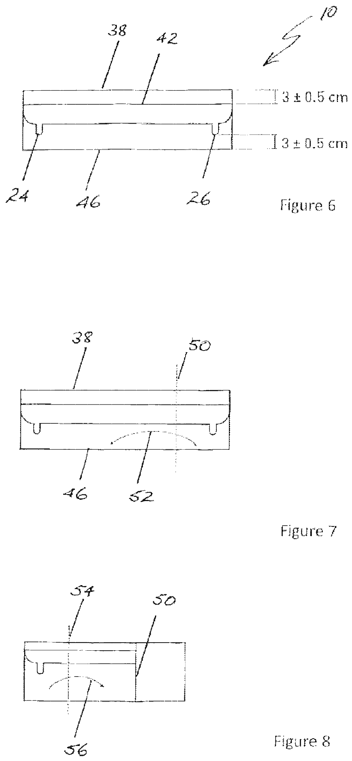

The result is shown in Figure 6. Tabs 24 and 26 are now visible. In this embodiment, the distance between third fold line 42 and second fold line 38 should be about 2.5 to 3.5 cm. The distance between fourth fold line 46 and the extremity of tabs 24 and 26 should also be about 2.5 to 3.5 cm. Each of fold lines 42 and 46 should be adjusted to this range if needed, to assist in preserving the sterile field of the wrap.

Figure 7 shows the location of first perpendicular fold line 50, which is substantially perpendicular to the first to fourth fold lines 34, 38, 42 and 46. Wrap 10 is folded at fold line 50, in the direction of arrow 52.

The result is shown in Figure 8, which also shows the location of second perpendicular fold line 54. Wrap 10 is folded at fold line 54, in the direction of arrow 56.

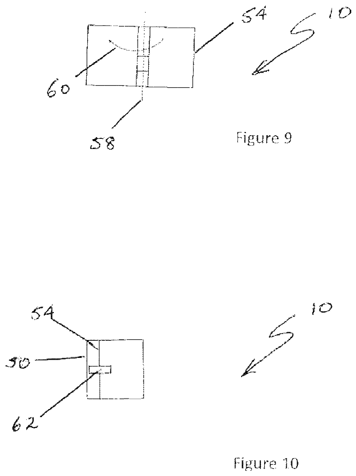

The result is shown in Figure 9, which also shows the location of third perpendicular fold line 58. Wrap 10 is folded at fold line 58, in the direction of arrow 60.

Tray 30 is now wrapped as shown in Figure 10. A sticker 62 is placed across fold line 54 to hold tray 30 in the wrapped state.

Tray 30 wrapped by wrap 10 is then sterilised and contained within a plastic film, and stored until ready for use.

To use, the plastic film and the sticker 62 are removed or torn. To open wrap 10 and expose tray 30, the user reverses the steps shown in Figures 8 to 10, until wrap 10 is in the configuration shown in Figure 7. The user then takes hold of each of tabs 24 and 26 and pulls them away from tray 30. Wrap 10 is now in the configuration shown in Figure 4.

Next, the user takes hold of tabs 20 and 22 and pulls them away from tray 30. Tray 30 is now unwrapped, as shown in Figure 1 or 2.

Because wrap 10 can be unwrapped using designated tabs 20, 22, 24 and 26, avoiding difficulty or confusion in locating corners as in prior art wraps, and because unwrapping can be carried out more rapidly, the wrap 10 represents an improvement over prior art wraps.

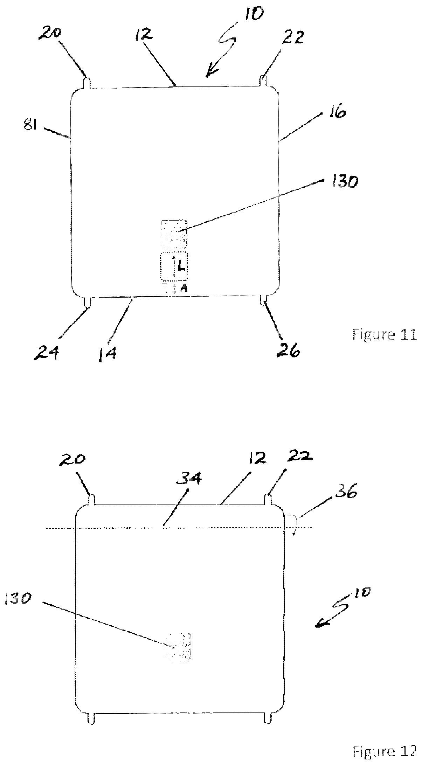

In the second embodiment in Figures 11 to 21, the wrap is the same as that in the first embodiment and the same labels are used to denote the same features. The method of wrapping is modified in that it includes the optional folding step (b1).

In the second embodiment, tray 130 is regarded as small and is 90mm long and 90mm wide. As shown in Figure 11, tray 130 is placed on wrap 10 approximately centrally between edge 16 and edge 18. Tray 130 is inboard of second edge 14 by about the length L of tray 130 plus about 2cm (indicated by arrow A).

Figure 12 shows first fold line 34, which is a small distance (between about 3 and 5 cm) inboard of first edge 12. Wrap 10 is folded under, in the direction of arrow 36, at first fold line 34, so that tabs 20 and 22 are under wrap 10, and the fold is flattened.

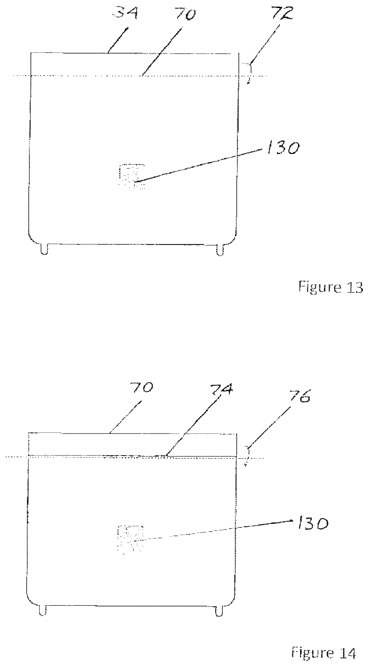

The result is shown in Figure 13, in which optional fold line 70 is indicated. Wrap 10 is then folded under, along fold line 70, as indicated by arrow 72. The distance between fold line 34 and fold line 70 is about 3 to 5 cm.

The result is shown in Figure 14, in which second optional fold line 74 is indicated. Wrap 10 is then folded under, along fold line 74, as indicated by arrow 76. The distance between fold line 70 and fold line 74 is about 3 to 5 cm.

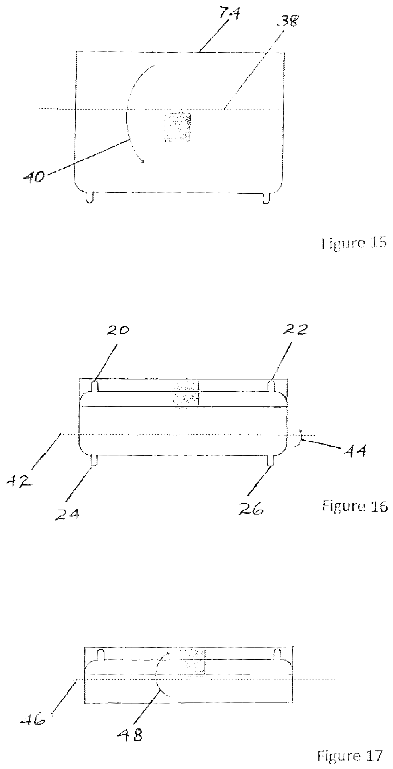

Figure 15 shows wrap 10 after folding along fold line 74 and indicating the location of second fold line 38. Wrap 10 is folded about second fold line 38, towards tray 130, in the direction of arrow 40. Second fold line 38 is located so that after folding tray 130 will be only just covered by wrap 10.

The result is shown in Figure 16. Tabs 20 and 22 are now visible. Wrap 10 is then folded at third fold line 42, which is a small distance (between about 4 and 6 cm) inboard of second edge 14, towards tray 130, in the direction of arrow 44, so that tabs 24 and 26 are under wrap 10. The fold is flattened.

The result is shown in Figure 17. Wrap 10 is then folded at fourth fold line 46, towards tray 130, in the direction of arrow 48. Fourth fold line 46 is located so that after folding tray 30 is further covered by wrap 10.

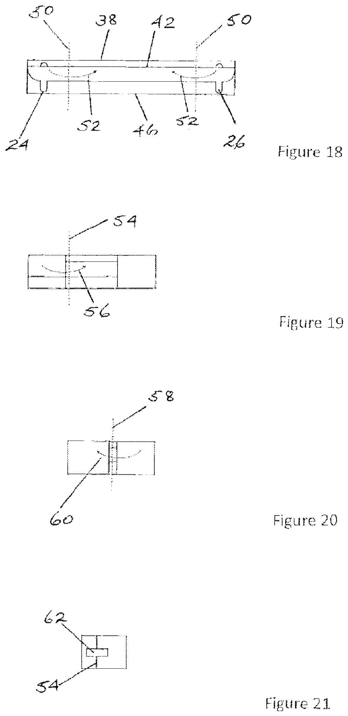

The result is shown in Figure 18. Tabs 24 and 26 are now visible. In this embodiment, the distance between third fold line 42 and second fold line 38 should be about 2.5 to 3.5 cm. The extremity of tabs 24 and 26 is about level with fourth fold line 46.

Figure 18 also shows the location of first perpendicular fold lines 50, which are substantially perpendicular to the first to fourth fold lines 34, 38, 42 and 46. Wrap 10 is folded at fold lines 50, in the direction of arrows 52.

The result is shown in Figure 19, which also shows the location of second perpendicular fold line 54. Wrap 10 is folded at fold line 54, in the direction of arrow 56.

The result is shown in Figure 20, which also shows the location of third perpendicular fold line 58. Wrap 10 is folded at fold line 58, in the direction of arrow 60.

Tray 130 is now wrapped as shown in Figure 21. A sticker 62 is placed across fold line 54 to hold tray 130 in the wrapped state.

Tray 130 wrapped by wrap 10 is then sterilised and contained within a plastic film, and stored until ready for use.

It will be appreciated by one skilled in the art that the embodiments described are not limiting on the scope of the invention and that modifications and variations may be made within the scope of the appended claims.

### Industrial Applicability

The wrap and methods of the present invention have many advantages. The unwrapping of a sterilised object can be achieved much more quickly and efficiently than at present. The likelihood of compromising asepsis is greatly reduced.

## Claims

1. A wrap (10) for wrapping an object (30,130) to be sterilised, the wrap (10) having four edges (12, 14, 16, 18) bound by a periphery, with a first edge (12) on one side of the periphery and a second edge (14) on a side of the periphery opposite to the first side (12), wherein:
- the wrap (10) has at least a first tab (20) forming part of the periphery of the first edge (12) and at least a second tab (26) forming part of the periphery of the second edge (14); and
- the first tab (20) is diagonally opposite the second tab (26).

2. The wrap (10) of claim 1, wherein the wrap (10) has a third tab (22) forming part of the periphery of the first edge (12) and a fourth tab (24) forming part of the periphery of the second edge (14), the third tab (22) being diagonally opposite the fourth tab (24).

3. The wrap (10) of claim 2, wherein the first and third tabs (20, 22) at the first edge (12) are spaced apart and the second and fourth tabs (24, 26) at the second edge (14) are spaced apart.

4. The wrap (10) of claim 3, wherein the first tab (20) is opposite the fourth tab (24) and the third tab (22) is opposite the second tab (26).

5. The wrap (10) of any one of claims 1 to 4, wherein each tab (20, 22, 24, 26) has parallel sides and a rounded end.

6. The wrap (10) of any one of claims 1 to 5, wherein each tab (20, 22, 24, 26) is integral with the wrap (10).

7. The wrap (10) of any one of claims 1 to 6, wherein the wrap (10) is rectilinear.

8. The wrap (10) of claim 7, wherein the wrap (10) has dimensions of up to about 1 metre by 1 metre.

9. The wrap (10) of claim 7, wherein the wrap (10) has dimensions of up to about 500 mm by 500 mm.

10. The wrap (10) of any one of claims 7 to 9 wherein the wrap (10) has rounded corners (28).

11. A method of wrapping an object (30, 130) to be sterilised using the wrap (10) as claimed in any one of claims 1 to 10, the method including the steps of:
(a) positioning the object (30, 130) on the wrap (10) so that the object (30, 130) is located approximately between the first edge (12) and the second edge (14);
(b) folding the wrap (10) at a first fold line (34) a small distance inboard of the first edge (12) so that the first tab (20) is under the wrap (10);
(c) folding the wrap (10) at a second fold line (38), inboard of the first fold line (34), towards the object (30, 130) so that the object is minimally covered by the wrap (10);
(d) folding the wrap (10) at a third fold line (42) a small distance inboard of the second edge (14) so that the second tab (26) is under the wrap (10);
(e) folding the wrap (10) at a fourth fold line (46), inboard of the third fold line (42), towards the object (30, 130) so that the object (30, 130) is further covered by the wrap (10); and
(f) folding the wrap (10) in a direction substantially perpendicular to the first to fourth fold lines (34, 38, 42, 46).

12. The method of claim 11, which includes step (b1) between step (b) and step (c), namely:
(b1) folding the wrap (10) towards the object (30, 130) at an optional fold line (70) a small distance inboard of the first fold line (34).

13. The method of claim 12, wherein step (b1) is repeated at least once.

14. The method of any one of claims claim 11 to 13, wherein the wrap (10) is further folded in directions substantially perpendicular to the first to fourth fold lines (34, 38, 42, 46).

15. The method of claim 14, wherein a sticker (62) is applied to the wrap (10) to hold the wrap (10) around the object (30, 130).

16. A wrapped object wherein the wrap (10) is as claimed in any one of claims 1 to 10.

17. A method of unwrapping the wrapped object as claimed in claim 16, wherein the wrap is folded at:
a first fold line (34) which is a small distance inboard of the first edge (12) so that the first tab (20) is under the wrap (10);
a second fold line (38) which is inboard of the first fold line (34) and towards the object (30, 130) so that the object is minimally covered by the wrap (10);
a third fold line (42) which is a small distance inboard of the second edge (14) so that the second tab (26) is under the wrap (10); and
a fourth line (46) which is inboard of the third fold line (42) and towards the object (30, 130) so that the object (30, 130) is further covered by the wrap (10);
the method including the steps of:
(a) removing or tearing any sticker holding the wrap around the wrapped object;
(b) unfolding the wrap in directions substantially perpendicular to the first to fourth fold lines;
(c) using the or each second tab to pull the wrap away from the folded object; and
(d) using the or each first tab to pull the wrap away from the folded object.

## Patentansprüche

1. Hülle (10) zum Umhüllen eines zu sterilisierenden Objekts (30, 130), wobei die Hülle (10) vier Kanten (12, 14, 16, 18) aufweist, die von einer Peripherie begrenzt werden, wobei sich eine erste Kante (12) auf einer Seite der Peripherie und eine zweite Kante (14) auf einer der ersten Seite (12) gegenüberliegenden Seite der Peripherie befindet, wobei:
- die Hülle (10) mindestens eine erste Lasche (20), die Teil der Peripherie der ersten Kante (12) bildet, und mindestens eine zweite Lasche (26), die Teil der Peripherie der zweiten Kante (14) bildet, aufweist; und
- die erste Lasche (20) der zweiten Lasche (26) diagonal gegenüber ist.

2. Hülle (10) nach Anspruch 1, wobei die Hülle (10) eine dritte Lasche (22), die Teil der Peripherie der ersten Kante (12) bildet, und eine vierte Lasche (24), die Teil der Peripherie der zweiten Kante (14) bildet, aufweist, wobei die dritte Lasche (22) diagonal gegenüber der vierten Lasche (24) ist.

3. Hülle (10) nach Anspruch 2, wobei die erste und dritte Lasche (20, 22) an der ersten Kante (12) voneinander beabstandet sind und die zweite und vierte Lasche (24, 26) an der zweiten Kante (14) voneinander beabstandet sind.

4. Hülle (10) nach Anspruch 3, wobei die erste Lasche (20) gegenüber der vierten Lasche (24) ist und die dritte Lasche (22) gegenüber der zweiten Lasche (26) ist.

5. Hülle (10) nach einem der Ansprüche 1 bis 4, wobei jede Lasche (20, 22, 24, 26) parallele Seiten und ein abgerundetes Ende hat.

6. Hülle (10) nach einem der Ansprüche 1 bis 5, wobei jede Lasche (20, 22, 24, 26) einstückig mit der Hülle (10) ist.

7. Hülle (10) nach einem der Ansprüche 1 bis 6, wobei die Hülle (10) geradlinig ist.

8. Hülle (10) nach Anspruch 7, wobei die Hülle (10) Abmessungen von bis zu 1 Meter mal 1 Meter aufweist.

9. Hülle (10) nach Anspruch 7, wobei die Hülle (10) Abmessungen von bis zu 500 mm mal 500 mm.

10. Hülle (10) nach einem der Ansprüche 7 bis 9, wobei die Hülle (10) abgerundete Ecken (28) aufweist.

11. Verfahren zum Umhüllen eines zu sterilisierenden Objekts (30, 130) mithilfe der Hülle (10) nach einem der Ansprüche 1 bis 10, wobei das Verfahren die folgenden Schritte aufweist:
(a) ein Positionieren des Objekts (30, 130) an der Hülle (10), so dass sich das Objekt (30, 130) ungefähr zwischen der ersten Kante (12) und der zweiten Kante (14) befindet;
(b) ein Falten der Hülle (10) an einer ersten Faltlinie (34), die zur ersten Kante (12) um einen geringen Abstand nach innen entfernt ist, so dass die erste Lasche (20) unter der Hülle (10) ist;
(c) ein Falten der Hülle (10) an einer zweiten Faltlinie (38), die nach innen zur ersten Faltlinie (34) ist, zum Objekt (30, 130), so dass das Objekt von der Hülle (10) minimal bedeckt wird;
(d) ein Falten der Hülle (10) an einer dritten Faltlinie (42), die zur zweiten Kante (14) um einen geringen Abstand nach innen entfernt ist, so dass die zweite Lasche (26) unter der Hülle (10) ist;
(e) ein Falten der Hülle (10) an einer vierten Faltlinie (46), die nach innen zur ersten Faltlinie (42) ist, zum Objekt (30, 130), so dass das Objekt (30, 130) von der Hülle (10) weiter bedeckt wird; und
(f) ein Falten der Hülle (10) in einer Richtung, die im Wesentlichen rechtwinklig zu den ersten bis vierten Faltlinien (34, 38, 42, 46) ist.

12. Verfahren nach Anspruch 11, das einen Schritt (b1) zwischen Schritt (b) und Schritt (c) beinhaltet, und zwar:
(b1) ein Falten der Hülle (10) zum Objekt (30, 130) an einer optionalen Faltlinie (70) um einen geringen Abstand nach innen zur ersten Faltlinie (34).

13. Verfahren nach Anspruch 12, wobei Schritt (b1) mindestens einmal wiederholt wird.

14. Verfahren nach einem der Ansprüche 11 bis 13, wobei die Hülle (10) ferner in Richtungen gefaltet wird, die im Wesentlichen rechtwinklig zur ersten bis vierten Faltlinie (34, 38, 42, 46) sind.

15. Verfahren nach Anspruch 14, wobei ein Aufkleber (62) auf der Hülle (10) angebracht wird, um die Hülle (10) um das Objekt (30, 130) zu halten.

16. Ein umhülltes Objekt (10), bei dem die Hülle (10) so ist, wie nach einem der Ansprüche 1 bis 10 beansprucht.

17. Verfahren zum Enthüllen des umhüllten Objekts nach Anspruch 16, wobei die Hülle gefaltet wird an:
einer ersten Faltlinie (34), die zur ersten Kante (12) um einen geringen Abstand nach innen entfernt ist, so dass die erste Lasche (20) unter der Hülle (10) ist;
einer zweiten Faltlinie (38), die nach innen zur ersten Faltlinie (34) und zum Objekt (30, 130) ist, so dass das Objekt von der Hülle (10) minimal bedeckt wird;
einer dritten Faltlinie (42), die zur zweiten Kante (14) um einen geringen Abstand nach innen entfernt ist, so dass die zweite Lasche (26) unter der Hülle (10) ist; und
einer vierten Faltlinie (46), die nach innen zur ersten Faltlinie (42) und zum Objekt (30, 130) ist, so dass das Objekt (30, 130) von der Hülle (10) weiter bedeckt wird;
wobei das Verfahren die folgenden Schritte beinhaltet:
(a) ein Entfernen oder Reißen von Aufklebern, die die Hülle um das umhüllte Objekt halten;
(b) ein Entfalten der Hülle in Richtungen, die im Wesentlichen rechtwinklig zur ersten bis vierten Faltlinie sind;
(c) ein Verwenden der oder jeder zweiten Lasche, um die Hülle vom gefalteten Objekt wegzuziehen, und
(d) ein Verwenden der oder jeder ersten Lasche, um die Hülle vom gefalteten Objekt wegzuziehen.

## Revendications

1. Enveloppe (10) pour envelopper un objet (30, 130) à stériliser, l'enveloppe (10) ayant quatre bords (12, 14, 16, 18) reliés par une périphérie, avec un premier bord (12) sur un côté de la périphérie et un deuxième bord (14) sur un côté de la périphérie opposé au premier côté (12), dans laquelle :
- l'enveloppe (10) a au moins une première languette (20) faisant partie de la périphérie du premier bord (12) et au moins une deuxième languette (26) faisant partie de la périphérie du deuxième bord (14) ; et
- la première languette (20) est diagonalement opposée à la deuxième languette (26).

2. Enveloppe (10) selon la revendication 1, dans laquelle l'enveloppe (10) a une troisième languette (22) faisant partie de la périphérie du premier bord (12) et une quatrième languette (24) faisant partie de la périphérie du deuxième bord (14), la troisième languette (22) étant diagonalement opposée à la quatrième languette (24).

3. Enveloppe (10) selon la revendication 2, dans laquelle les première et troisième languettes (20, 22) du premier bord (12) sont espacées et les deuxième et quatrième languettes (24, 26) du deuxième bord (14) sont espacées.

4. Enveloppe (10) selon la revendication 3, dans laquelle la première languette (20) est opposée à la quatrième languette (24) et la troisième languette (22) est opposée à la deuxième languette (26).

5. Enveloppe (10) selon l'une quelconque des revendications 1 à 4, dans laquelle chaque languette (20, 22, 24, 26) a des côtés parallèles et une extrémité arrondie.

6. Enveloppe (10) selon l'une quelconque des revendications 1 à 5, dans laquelle chaque languette (20, 22, 24, 26) est solidaire de l'enveloppe (10).

7. Enveloppe (10) selon l'une quelconque des revendications 1 à 6, dans laquelle l'enveloppe (10) est rectiligne.

8. Enveloppe (10) selon la revendication 7, dans laquelle l'enveloppe (10) a des dimensions de jusqu'à environ 1 mètre par 1 mètre.

9. Enveloppe (10) selon la revendication 7, dans laquelle l'enveloppe (10) a des dimensions de jusqu'à environ 500 mm par 500 mm.

10. Enveloppe (10) selon l'une quelconque des revendications 7 à 9, dans laquelle l'enveloppe (10) a des coins arrondis (28).

11. Procédé pour envelopper un objet (30, 130) à stériliser à l'aide de l'enveloppe (10) selon l'une quelconque des revendications 1 à 10, le procédé incluant les étapes suivantes :
(a) le positionnement de l'objet (30, 130) sur l'enveloppe (10) de telle sorte que l'objet (30, 130) est approximativement entre le premier bord (12) et le deuxième bord (14) ;
(b) le pliage de l'enveloppe (10) au niveau d'une première ligne de pliage (34) à une petite distance à l'intérieur du premier bord (12) de telle sorte que la première languette (20) est sous l'enveloppe (10) ;
(c) le pliage de l'enveloppe (10) au niveau d'une deuxième ligne de pliage (38), à l'intérieur de la première ligne de pliage (34), vers l'objet (30, 130) de telle sorte que l'objet est minimalement recouvert par l'enveloppe (10) ;
(d) le pliage de l'enveloppe (10) au niveau d'une troisième ligne de pliage (42) à une petite distance à l'intérieur du deuxième bord (14) de telle sorte que la deuxième languette (26) est sous l'enveloppe (10) ;
(e) le pliage de l'enveloppe (10) au niveau d'une quatrième ligne de pliage (46), à l'intérieur de la troisième ligne de pliage (42), vers l'objet (30, 130) de telle sorte que l'objet (30, 130) est davantage recouvert par l'enveloppe (10) ; et
(f) le pliage de l'enveloppe (10) dans une direction essentiellement perpendiculaire aux première à quatrième lignes de pliage (34, 38, 42, 46).

12. Procédé selon la revendication 11, qui inclut l'étape (b1) entre l'étape (b) et l'étape (c), à savoir :
(b1) le pliage de l'enveloppe (10) vers l'objet (30, 130) au niveau d'une ligne de pliage facultative (70) à une petite distance à l'intérieur de la première ligne de pliage (34).

13. Procédé selon la revendication 12, dans lequel l'étape (b1) est répétée au moins une fois.

14. Procédé selon l'une quelconque des revendications 11 à 13, dans lequel l'enveloppe (10) est en outre pliée dans des directions essentiellement perpendiculaires aux première à quatrième lignes de pliage (34, 38, 42, 46).

15. Procédé selon la revendication 14, dans lequel un autocollant (62) est appliqué sur l'enveloppe (10) pour maintenir l'enveloppe (10) autour de l'objet (30, 130).

16. Objet enveloppé, dans lequel l'enveloppe (10) est selon l'une quelconque des revendications 1 à 10.

17. Procédé pour désenvelopper l'objet enveloppé selon la revendication 16, dans lequel l'enveloppe est pliée au niveau :
d'une première ligne de pliage (34) qui est à une petite distance à l'intérieur du premier bord (12) de telle sorte que la première languette (20) est sous l'enveloppe (10) ;
d'une deuxième ligne de pliage (38) qui est à l'intérieur de la première ligne de pliage (34) et vers l'objet (30, 130) de telle sorte que l'objet est minimalement recouvert par l'enveloppe (10) ;
d'une troisième ligne de pliage (42) qui est à une petite distance à l'intérieur du deuxième bord (14) de telle sorte que la deuxième languette (26) est sous l'enveloppe (10) ; et
d'une quatrième ligne (46) qui est à l'intérieur de la troisième ligne de pliage (42) et vers l'objet (30, 130) de telle sorte que l'objet (30, 130) est davantage recouvert par l'enveloppe (10) ;
le procédé incluant les étapes suivantes :
(a) l'enlèvement ou le déchirement de tout autocollant maintenant l'enveloppe autour de l'objet enveloppé ;
(b) le dépliage de **l'enveloppe** dans des directions essentiellement perpendiculaires aux première à quatrième lignes de pliage ;
(c) l'utilisation de la ou de chaque deuxième languette pour éloigner l'enveloppe de l'objet plié ; et
(d) l'utilisation de la ou de chaque première languette pour éloigner l'enveloppe de l'objet plié.
